# EUROPEAN PATENT APPLICATION

(11) **EP 1 190 709 A1**
(43) Date of publication of application: **27.03.2002**
(21) Application number: 00120652.3
(22) Date of filing: 21.09.2000
(51) Int. Cl.: A61K 31/00, A61K 31/475, A61K 38/10, A61P 27/16

(54) **Treatment of tinnitus**

(71) Applicant: Tinnitus Forschungs- und Entwicklungs GmbH, 80333 München (DE)
(72) Inventor: Ruppersberg, J. Peter, Prof.Dr., 72072 Tübingen (DE); Fakler, Bernd, 72076 Tübingen (DE)
(74) Representative: Patentanwälte Ruff, Wilhelm, Beier, Dauster & Partner

(57) **Abstract**

The invention relates to the use of substances which are at least partly blocking one ionotropic acetylcholine receptor of the inner ear and/or a calcium-activated potassium channel functionally associated with said acetylcholine receptor of the inner ear for the manufacturing of a pharmaceutical composition or medicament for the treatment of tinnitus.

## Description

The invention relates to the use of substances for the manufacturing of a pharmaceutical composition or medicament for the treatment of tinnitus.

Two main cell types are involved in the processing of sound. The inner hair cells which work as transducers to the central nervous system (CNS) and the outer hair cells which are electro-mechanical amplifiers for sound on the basis of a loudspeaker-like-mechanism. The outer hair cells (OHC) receive synaptic input from the superior olive that controls cochlear afferent activity by inhibiting the fast voltage-dependent amplification mechanism provided by these sensory cells (Guinan, J. 1996. Dallos, P., Popper, N. and Fay, R. eds. New York: Springer, 435-502). This fast synaptic inhibition is generally mediated by a hyperpolarizing chlorid conductance through different receptors activated by transmitters released from the presynapse (Alger, B.E. 1991. Ann. NY Acad. Sci. 627, 249-263; Betz, H., Kuhse, J., Schmieden, V., Laube, B., Kirsch, J. and Harvey, R.J. 1999. Ann. NY Acad. Sci. 868, 667-676). How this synaptic inhibition in OHCs works is still unclear. What is known is that neuronal acetylcholine receptors (nAChR) in OHCs supply the prosynaptic cell with calcium (Ca²⁺) that initiates an inhibitory hyperpolarization by opening a calcium²⁺-activated potassium (SK) channel (Oliver, D., Klöcker, N., Schluck, J., Baukrowitz, T., Ruppersberg, J.P. and Fakler, B. 2000. Neuron 26, 595-601).

Damages of the inner ear occur e.g. by traumatic, hypoxic and toxic influences. The main reasons for that damages are noise, sudden hearing loss and drugs e.g. antibiotics or cytostatics. Diseases or disturbances in which subjective noise in the ear, a so-called tinnitus occurs, are widespread. It is estimated that in Germany alone roughly six million people suffer from tinnitus. In roughly 800.000 cases the tinnitus is so pronounced that these patients need intensive treatment by a physician, due to the patient being seriously handicapped by tormenting ear noise.

In the case of tinnitus different medical therapies have been proposed hitherto. These include, in addition to the use of anaesthetica, the application of lidocaine-type antiarrhythmica or anticonvulsiva, or infusion therapy. However, in most cases treatments of this kind fail to bring satisfactory results.

In the European Patent 0759295 it was shown, that adamantane derivatives can be successfully used for the treatment of tinnitus which is associated with a so-called positive recruitment and/or a reduction or a failure of otoacustic emissions. However, the molecular mechanism how adamantane derivative works was still unknown, although there was evidence that receptors present on the outer hair cells may be involved. In the publication of Oliver et al. (Oliver, D. et al., 2000, see above) it was shown that the hyperpolarization of OHC cells is mediated by SK2 channels and that it occurs rapidly, explaining a possible mechanism for the generation of fast inhibitory synaptic transmission.

The object of the invention is to find new targets and to make available new substance classes for the treatment of tinnitus by definitely explaining the molecular mechanism involved in the signaltransmission of the OHCs which might be a reason for the pathomechanism of tinnitus. This object is solved by the subject-matter of the independent claims 1 and 10. Preferred embodiments are given in the dependent claims 2 to 9 and 11 to 17. The wording of all these claims is hereby incorporated into the content of the description by reference.

Surprisingly it was found that neuronal acetylcholine receptors of the inner ear comprise alpha9- and alpha10-subunits. Therefore, there is a alpha9/alpha10 heteromeric acetylcholine receptor. Via such acetylcholine receptors the hyperpolarization of outer hair cells (OHCs) occurs and it is mediated by SK2-channels. The acetylcholine receptor can be blocked efficiently by memantine (3.5-dimethyl-1-adamantanamine) and other substances and therefore an inactivation of the OHCs occurs. Furthermore, it was found that this ionotropic acetylcholine receptor is functionally associated with a calcium-activated potassium channel. Blocking of this channel can also lead to the inactivation of the OHCs. These new findings make it possible to use the acetylcholine receptor and/or the SK-channel as a new target for the treatment of tinnitus. Accordingly, new substance classes can be used blocking the acetylcholine receptor and/or the calcium-activated potassium channel.

The potential pathomechanism of tinnitus may also be explained by these findings. The OHC may physiologically or pathologically generate the psychical impression of sound by undergoing mechanical movements which are transmitted to the inner hair cells and through those propagated to the CNS. Such movements are caused by membrane potential changes caused either by sound or by the efferent synapse of the OHC. This synapse causes membrane potential changes which are not associated with real sound but interpreted by the CNS as pathological sound (tinnitus). As nAChR and the calcium-activated potassium channel are involved in this signal transmission of this synapse, any potent inhibitor of the signal transmission might inhibit tinnitus.

According to the invention, at least one substance is used for the manufacturing of a pharmaceutical composition or medicament for the treatment of tinnitus, wherein said substance is at least partly blocking at least one ionotropic acetylcholine receptor of the inner ear and wherein that substance is not an adamantane derivative according to the following formula 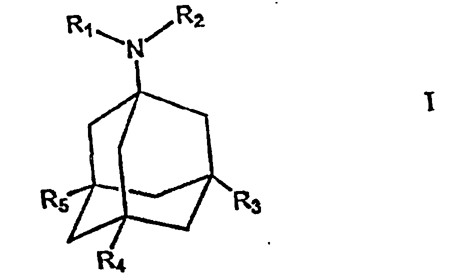 where R₁ and R₂
- are the same or different, and include hydrogen or straight or branched chain alkyl groups having 1 to 6 C atoms, or
- together with the N atom present a heterocyclic group having 5 or 6 ring atoms,

where R₃ and R₄ are the same or different, and include hydrogen, straight, or branched chain alkyl groups having 1 to 6 C atoms, cycloalkyl groups having 5 to 6 C atoms, or phenyl, and
where R₅ is hydrogen or a straight or branched chain alkyl group having 1 to 6 C atoms.

The substance can optionally be used in the form of its pharmaceutically acceptable salts and/or optionally together with a pharmaceutically carrier.

According to the invention, this ionotropic acetylcholine receptor of the inner ear comprises at least one so-called alpha9-subunit. As an alternative or preferably in addition the receptor comprises at least one so-called alpha10-subunit. Preferably the receptor is functionally associated with at least one calcium-activated potassium channel. This calcium-activated potassium channel is preferably of SK (small conductance) subtype, wherein preferably that SK potassium channel is of the SK2 subtype.

According to the invention the substance used for the treatment of tinnitus can be a strychnine derivative, a peptide, preferably a polypeptide, or a polynucleotide encoding such peptide, preferably a polypeptide. Furthermore, the substance can be a small molecular compound, preferably a small molecular compound with a molecular weight (MW) < 1000.

According to further embodiments the invention comprises further the use of a substance for the manufacturing of a pharmaceutical composition or medicament for the treatment of tinnitus, wherein said substance is at least partly blocking at least one calcium-activated potassium channel functionally associated with an ionotropic acetylcholine receptor of the inner ear and wherein that substance is not an adamantane derivative according to the following formula 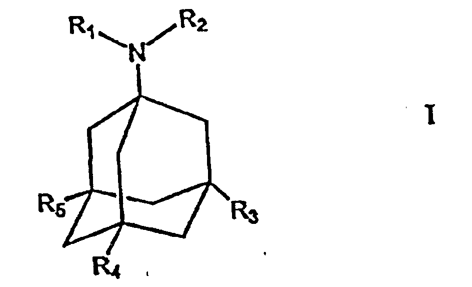 where R₁ and R₂
- are the same or different, and include hydrogen or straight or branched chain alkyl groups having 1 to 6 C atoms, or
- together with the N atom present a heterocyclic group having 5 or 6 ring atoms,

where R₃ and R₄ are the same or different, and include hydrogen, straight, or branched chain alkyl groups having 1 to 6 C atoms, cycloalkyl groups having 5 to 6 C atoms, or phenyl, and
where R₅ is hydrogen or a straight or branched chain alkyl group having 1 to 6 C atoms.

As described above, the substance can optionally be used in the form of its pharmaceutically acceptable salts and/or together with a pharmaceutically carrier.

The ionotropic acetylcholine receptor which is functionally associated with the calcium-activated potassium channel comprises according to these embodiments of the invention at least one so-called alpha9-subunit. As an alternative or preferably in addition this acetylcholine receptor comprises at least one so-called alpha10-subunit. The calcium-activated potassium channel is preferably of the SK subtype, wherein preferably said SK potassium channel is of the SK2 subtype.

Finally, according to the invention the substance which at least partly blocks at least one calcium-activated potassium channel defined as above can be a peptide, preferably a polypeptide. This polypeptide can be apamine, which preferably blocks this channel, but also even up to now unknown compounds which block this channel are claimed for the inventive use. The substance can also be a polynucleotide encoding such a peptide, preferably polypeptide, or it can be a small molecular compound, preferably a small molecular compound with a molecular weight (MW) < 1000.

The described features and further features of the invention can be gathered from the following description of preferred embodiments in conjunction with the subclaims. The individual features can be implemented seperately or in the form of subcombinations.

### MATERIALS AND METHODS

### Electrophysiology on OHCs

Organs of Corti were dissected from the cochleae of Wistar rats (11-24 days old) as described previously (Oliver, D. and Fakler, B. 1999. J. Physiol. (Lond.) 519 pt. 3, 791-800; Oliver, D. et al., 2000, see above). The electrophysiology on OHCs was also described previously (Oliver, D. et al., 2000, see above).

### Electrophysiology on heterologously expressed proteins

In vitro mRNA synthesis and oocyte injections were performed as previously described (Fakler, B. Brandle, U., Glowatzki, E., Weidemann, S., Zenner, H.P. and Ruppersberg, J.P. 1995. Cell 80, 149-154; Oliver, D. et al., 2000, see above). The electrophysiology on oocytes was also performed as described previously (Oliver, D. et al., 2000, see above).

### Figure legends:

Fig. 1: (A) Model for the inhibitory synaptic transmission at OHCs as derived from experiments with isolated cells. (B) Inhibitory postsynaptic currents (IPSCs) were inhibited by strychnine and apamine.

Fig. 2: Block of alpha9/alpha10 SK2 current responses to acetylcholine in Xenopus oocytes by strychnine.

### Experiment 1:

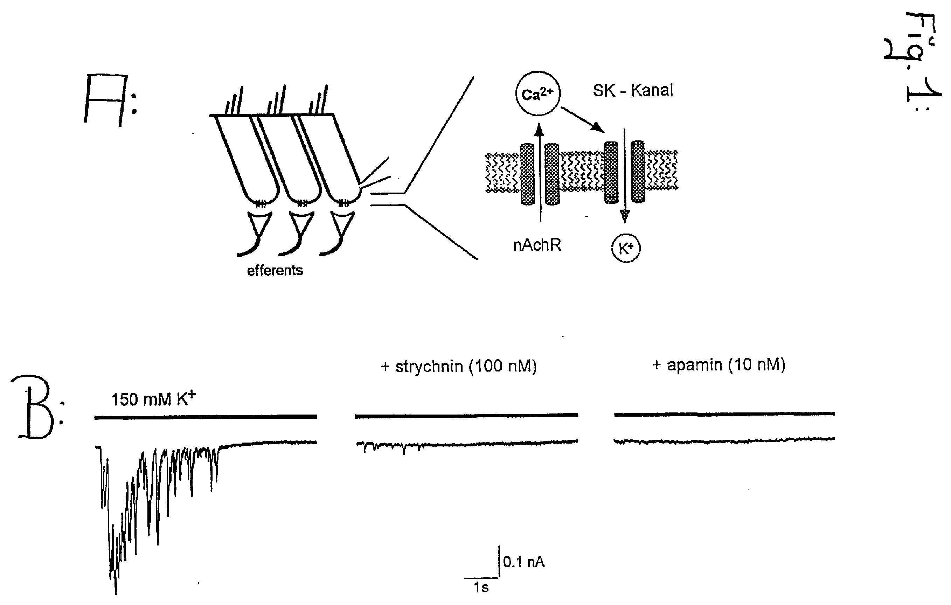

In Fig. 1A, a model is shown of how the inhibitory transmission at OHCs occurs. Ca²⁺ entering the postsynapse via the transmitter-activated nAChR opens SK channels and thus results in hyperpolarizing K⁺ currents (Art, J.J., Fettiplace, R. and Fuchs, P.A. 1984. J. Physiol. (Lond.) 356, 525-550; Yuhas, W.A. and Fuchs, P.A. 1999. J. Comp. Physiol. 18, 455-462).

In Fig 1B, postsynaptic currents were recorded from OHCs in voltage-clamp experiments when cells were held at -64 mV and the whole Corti preparation was superfused with high extracellular K⁺ (150 mM) to depolarize the presynapse. Application of high K⁺ and toxins as indicated by horizontal bars: current and time scaling as indicated.

Consistent with the model presented in Fig. 1A, the postsynaptic currents were inhibited by application of either the acetylcholine blocker strychnine (Elgoyhen, A.B., Johnson, D.S., Boutler, J., Vetter, D.E. and Heinemann, S. 1994. Cell 79, 705-715) or the SK channel blocker apamine (complete inhibition at 100 nM and 10 nM, respectively; Fig. 1B).

### Experiment 2:

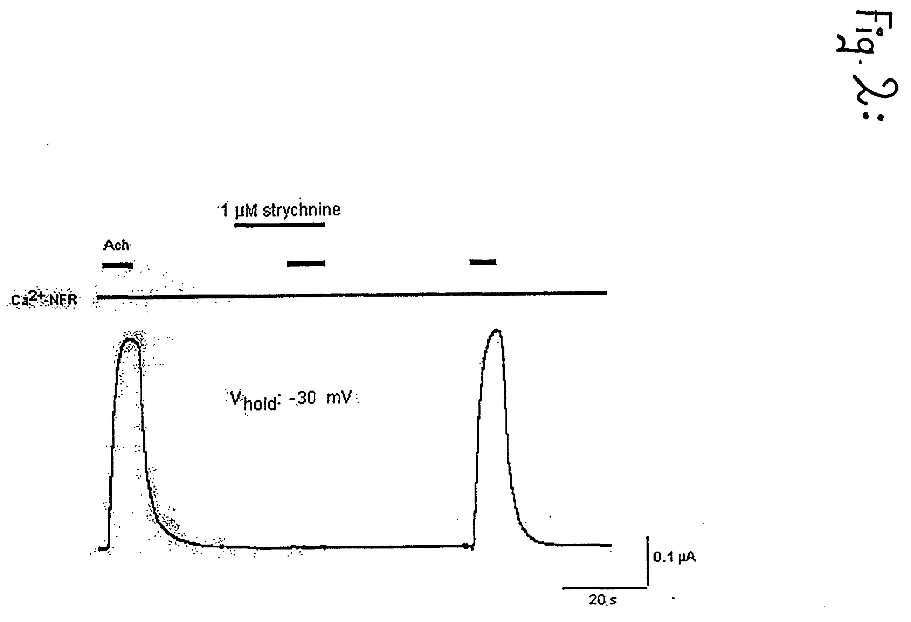

For further characterization of strychnine treatment on the nAChR, the mRNA of alpha9/alpha10 subunits of the acetylcholine receptor were injected in oocytes of Xexopus laevis. After expression of these subunits, they were investigated by treatment with strychnine in voltage-clamp experiment. The cells were held at -30 mV. Current and time scaling are as indicated. As is shown in Fig. 2, similar results were obtained as in Fig. 1. Consistent with the model shown above, the current responses at the acetylcholine receptor could be blocked reversibly by application with the acetylcholine blocker strychnine. A complete inhibition occurs at 1 µM, and after washout of strychnine for 3 min. the receptor was again functionally active, indicating a reversible block of the receptor.

## Claims

1. Use of a substance for the manufacturing of a pharmaceutical composition or medicament for the treatment of tinnitus, wherein said substance is at least partly blocking at least one ionotropic acetylcholine receptor of the inner ear and wherein said substance is not an adamantane derivative according to the following formula 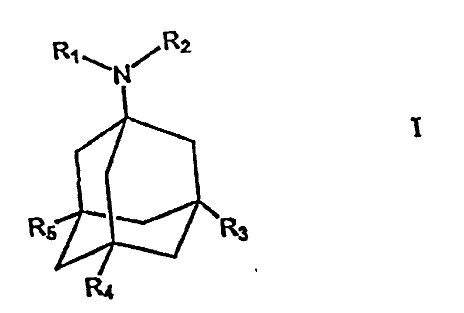 where R₁ and R₂
- are the same or different, and include hydrogen or straight or branched chain alkyl groups having 1 to 6 C atoms, or
- together with the N atom present a heterocyclic group having 5 or 6 ring atoms,
where R₃ and R₄ are the same or different, and include hydrogen, straight or branched chain alkyl groups having 1 to 6 C atoms, cycloalkyl groups having 5 to 6 C atoms, or phenyl, and
where R₅ is hydrogen or a straight or branched chain alkyl group having 1 to 6 C atoms.

2. Use according to claim 1, **characterized in that** said acetylcholine receptor comprises at least one alpha9-subunit.

3. Use according to claim 1 or claim 2, **characterized in that** said acetylcholine receptor comprises at least one alpha10-subunit.

4. Use according to one of the preceding claims, **characterized in that** said acetylcholine receptor is functionally associated with at least one calcium-activated potassium channel.

5. Use according to claim 4, **characterized in that** said calcium-activated potassium channel is of SK subtype, wherein preferably said SK potassium channel is SK2.

6. Use according to one of the preceding claims, **characterized in that** said substance is a strychnine derivative.

7. Use according to one of claims 1 to 5, **characterized in that** said substance is a peptide, preferably a polypeptide.

8. Use according to one of claims 1 to 5, **characterized in that** said substance is a polynucleotide encoding a peptide, preferably a polypeptide, of claim 7.

9. Use according to one of the preceding claims, **characterized in that** said substance is a small molecular compound, preferably a small molecular compound with a molecular weight (MW) < 1000.

10. Use of a substance for the manufacturing of a pharmaceutical composition or medicament for the treatment of tinnitus, wherein said substance is at least partly blocking at least one calcium-activated potassium channel functionally associated with an ionotropic acetylcholine receptor of the inner ear and wherein said substance is not an adamantane derivative according to the following formula 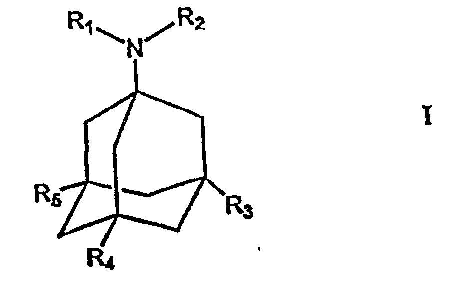 where R₁ and R₂
- are the same or different, and include hydrogen or straight or branched chain alkyl groups having 1 to 6 C atoms, or
- together with the N atom present a heterocyclic group having 5 or 6 ring atoms,
where R₃ and R₄ are the same or different, and include hydrogen, straight or branched chain alkyl groups having 1 to 6 C atoms, cycloalkyl groups having 5 to 6 C atoms, or phenyl, and
where R₅ is hydrogen or a straight or branched chain alkyl group having 1 to 6 C atoms.

11. Use according to claim 10, **characterized in that** said acetylcholine receptor comprises at least one alpha9-subunit.

12. Use according to claim 10 or claim 11, **characterized in that** said acetylcholine receptor comprises at least one alpha10-subunit.

13. Use according to one of claims 10 to 12, **characterized in that** said calcium-activated potassium channel is of SK subtype, wherein preferably said SK potassium channel is SK2.

14. Use according to one of claims 10 to 13, **characterized in that** said substance is a peptide, preferably a polypeptide.

15. Use according to claim 14, **characterized in that** said peptide is apamine.

16. Use according to one of claims 10 to 13, **characterized in that** said substance is a polynucleotide encoding a peptide, preferably a polypeptide, of claim 14.

17. Use according to one of claims 10 to 16, **characterized in that** said substance is a small molecular compound, preferably a small molecular compound with a molecular weight (MW) < 1000.
